## Europäisches Patentamt

## European Patent Office

## Office européen des brevets

(11) Publication number: **0 143 517**
**B1**

(12) **EUROPEAN PATENT SPECIFICATION**

(45) Date of publication of patent specification: **05.04.89**

(51) Int. Cl.⁴: **A 61 M 5/00**

(21) Application number: **84306023.7**

(22) Date of filing: **03.09.84**

(54) Implantable parenteral hyperalimentation catheter system.

(30) Priority: **02.09.83 US 528990**

(43) Date of publication of application:
**05.06.85 Bulletin 85/23**

(45) Publication of the grant of the patent:
**05.04.89 Bulletin 89/14**

(84) Designated Contracting States:
**DE FR GB IT SE**

(56) References cited:
**EP-A-0 066 667**
**GB-A-2 073 027**
**US-A-4 108 173**
**US-A-4 321 914**
**US-A-4 417 888**

(73) Proprietor: **Minntech Corporation**
**14905 28th Avenue North**
**Minneapolis Minnesota 55441 (US)**

(72) Inventor: **Martinez, F. Jesus**
**2625 Jewel Lane**
**Plymouth Minnesota 55447 (US)**
Inventor: **Fuller, Larry e.**
**13720 Summit Lane**
**Minnetonka Minnesota 55434 (US)**
Inventor: **Cosentino, Louis C.**
**2435 Holly Lane North**
**Wayzata Minnesota 55447 (US)**

(74) Representative: **Marlow, Nicholas Simon et al**
**Reddie & Grose 16, Theobalds Road**
**London WC1X 8PL (GB)**

## Description

### Background of the Invention

#### 1. Field of the Invention

This invention relates to the field of implantable percutaneous devices and more particularly to the field of devices used in dispensing nutrients intraveneously into the heart or central venous system.

#### 2. Description of the Prior Art

Parenteral hyperalimentation (hereinafter referred to as PH) involves an intraveneous infusion of hypertonic solutions of dextrose, amino acids, vitamins and minerals through a venous catheter to support normal growth and development in a child and positive nitrogen balance with weight gain in a surgically stressed adult or in adults unable to digest food properly. PH is useful whenever the patient is unable to maintain an adequate oral intake for more than seven days, or has an inpaired intestinal absorption detrimental to anticipated surgery or convalescence.

Placement of hyperalimentation lines in the right atrium or central large veins through the subclavian vein has become a relatively common technique.

Simple polyethelene catheters are not suitable for prolonged periods because of possible thrombosis. Special silicone catheters known as the Broviac-Scribner or the wider bore Hicknan catheters have been utilized for long term PH. Such catheters are about 90 centimeters long and are composed generally of a thin, flexible intravascular segment with a thickened extravascular segment which has a Dacron® cuff in its midpoint. The extravascular Dacron® portion lies in a subcutaneous tunnel cut through the skin. The Dacron® cuff allows for the ingrowth of fibrous tissue. The ingrowth helps fix the catheter in place and acts as a barrier to the passage of bacteria through the subcutaneous track.

The extravascular portion of the catheters are tunneled under the skin and brought out in the fourth or fifth interspace, just lateral to the sternum at a site where the patient can see and care for the catheter. Catheters of the prior art utilises a plastic Luer cap so that the line can be heparinised and sealed between infusions. The catheters are inserted through a venotomy into the cephalic vein or jugular vein, or any other vein which allows access to the superior vena cava. Nutrients may then be supplied directly into the right auricle of the heart. The nutrients are thereby placed in a position of high blood flow and may be passed to all areas of the body which need nutrition, unlike the administration of nutrients into a remote vein or artery.

It has been found that indwelling catheters are major sites of colonisation of microorganisms, especially by skin flora, such as Staphylococcus epidermidis, diptheroids and Bacillus sp. Common infection pathways are through the interior of the catheter and along the exterior surfaces of the catheter itself.

In EP—A—0 078 565 there is disclosed a percutaneous implant device comprising a substantially rigid tubular percutaneous body of biologically compatible material which extends through the skin when implanted; a flexible subcutaneous catheter joined to the tubular percutaneous body for providing fluid communication between the body exterior and the right auricle of the heart; carrier means including a probe carrier for providing fluid communication from the nutrient supply tube to said catheter; and porous tissue ingrowth means for anchoring the device to the body.

The present invention is characterised in that the porous tissue ingrowth means comprise a tissue ingrowth sleeve comprising a porous collar of polytetrafluoroethylene overlaying the portion of the tubular percutaneous body to be embedded in the epidermis and an adjacent porous collar of polyethylene terephthalate being of sufficient length to match the thickness of the dermis; and a sliding wing of porous tissue ingrowth material encircling part of, and slidably positionable on, the subcutaneous catheter.

The tubular body extending through the skin after implantation provides a fluid passageway for accessing the heart. The sleeve length is sufficient to pass through the epidermis and dermis layers of the skin and has sufficient porosity to permit tissue ingrowth therein. It is believed that the use of a polytetrafluoroethylene polymer to contact the epidermis provides better tissue ingrowth in the outermost non-vascular epiphelial layer of skin.

The catheter is preferably held in place through the tubular body by forcing the catheter over raised lips on the end of the tubular body. The sliding wing may be moved in either direction by the surgeon to position the catheter in place and anchor the catheter into position once the tissue has ingrown over the sliding wing.

In the preferred embodiment, an elastomeric septum is positioned in sealing relationship in the tubular body and is compressed between a support extension within the tubular body and a pressure plate above the septum. The septum is provided with preformed openings therethrough to provide a resealable pathway for a needle.

A locking ring holds the septum member and pressure plate within the tubular body. An excutaneous cavity is defined above the septum within the tubular body.

The implant of the invention is inserted following any of the established procedures for implanting hyperalimentation devices. Generally, the cephalic vein is isolated in a deltopectoral groove, the distal segment is ligated after an incision is made below the acromion. From that incision, a long clamp is directed down through the subcutaneous tissue to construct a subcutaneous tunnel. The tip is then exteriorized through a small incision and the catheter is drawn back into the tunnel. The catheter is then positioned in the tunnel so that the sliding wing lies in the tunnel between the two incisions and in an intercostal space. The sliding wing is easily felt for positioning, and may be seen readily through a fluoroscope.

The remainder of the catheter tubing is then cut to fit the anatomy of the patient by plotting the distance the catheter must travel to follow the course of the cephalic vein to the subclavian vein and the superior vena cava and into the entrance of the right atrium, at which level one wants the tip of the catheter to lie. The incisions are then closed and the implant is held fixedly in place at the skin surface due to tissue ingrowth at the access body and to tissue ingrowth around the sliding wing positioned within the subcutaneous tunnel. The use of a polytetrafluoroethylene sleeve through the epidermis and a polyethylene terephthalate sleeve through the dermis or vascular layer of skin tissue provides excellent attachment of the device to the skin. There is no need for a subcutaneous stabilizing flange. Slight movement of the access body does not disturb the position of the catheter tip within the right auricle due to the embedment of the sliding wing within the tissue. An exterior pull on the access body may cause the catheter between the sliding wing and the access body to move slightly, but does not cause the tip of the catheter within the right auricle to move. This avoids myocardial irritability, valvular trauma, or even perforation with cardiac tamponade.

Nutrients are introduced through the implant which is secured to the body through the use of the access set. The access set includes a rigid, single probe carrier. Tubing is connected to one side of the carrier which then leads to a source of nutrients. The nutrients typically will be supplied through the regulation of a pump or by gravity feed from the nutrient source. The other end of the carrier is attached to a short shell of 11 gauge tubing which has holes punched through its walls adjacent to its closed end and/or a hole punched at the rounded tip end. The short shell is of sufficient length such that when the carrier is inserted into the excutaneous space defined by the access body, the short shell passes through the slitted septum, such that liquid can pass through the holes in the shell and into the catheter.

The single probe carrier is held to the rigid tubular access body by a flexible silicone carrier cap which is constructed and arranged such that the single probe carrier is snap fitted into place within the carrier cap. The carrier cap, single probe cap tubing and short shell are then linked to the rigid tubular access body by snap fitting the carrier cap over a circumferential ridge on the rigid tubular access body such that the access set is held within position.

Ambulatory patients may disconnect the access set from the device once the nutrient "feeding" or chemotherapy is completed, thereby giving the patient increased mobility. When not in use, the device's internal excutaneous cavity is maintained sterile by the addition of a solution such as Betadine®, as polyvinyl pyrolidone/iodine solution, sold by Purdue Frederick Co. of Norwalk, Connecticut, held by a sponge in the device's cavity. The cavity is hermetically sealed from the environment by a tab cap.

The unique design of the invention allows the introduction of nutrients, chemotherapy agents or other fluids directly into a large vein where the fluids are transported throughout the body. Delivery into veins with a high flow rate minimizes the burning sensation associated with chemical introduction in smaller veins.

Brief Description of the Drawings

The detailed description of the invention, including its preferred embodiment, is hereinafter described with specific reference being made with the drawings in which:

Fig. 1 is a cross-sectional view of the device of the invention showing the skin line;

Fig. 2 is an exploded pictorial view of the device in accordance with the invention, and

Fig. 3 is an enlarged partial side elevational view of the device and tab cap with portions cut away.

Detailed Description of the Invention.

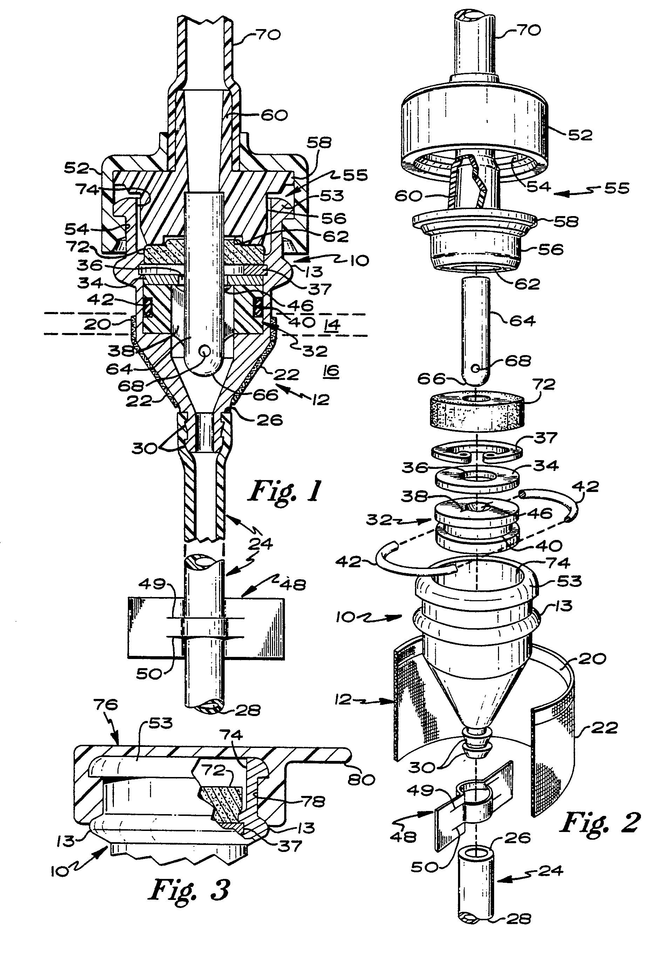

A preferred embodiment of the invention is shown in Figs. 1, 2, and 3. A rigid tubular percutaneous access body 10 and attached flexible sleeve member 12 is implanted through epidermis 14 and dermis 16. Rigid tubular body 10 is preferably made of titanium which may be coated with vapor-deposited carbon or other biocompatible coatings. Alternatively, body 10 may remain uncoated. Body 10 is preferably about 0.8 of an inch long (2.0 cm) with about 0.25 inches (6mm) of body 10 extending above the skin surface.

Tissue ingrowth media is affixed to the exterior of body 10 as a porous flexible sleeve member or cuff 12. Flexible sleeve member 12 serves as a tissue ingrowth media which stabilizes the implant and prevents its extrusion and minimizes bacterial penetration through the skin exit site. Flexible sleeve 12 preferably extends from 2—3 millimeters below raised lip 13 of body 10 and terminates where the catheter portion of the device begins. Flexible sleeve 12 is preferably made of a material such as the expanded polyethylene terephthalate sold under the trademark Dacron®, by E I. DuPont de Nemours of Wilmington, Delaware.

It has been found that the outer, nonvascular layer of the skin composed of epithelial tissue has different ingrowth characteristics from the underlying vascular connective tissue of the skin. Surprisingly better ingrowth has been found when a flexible sleeve portion 20, which contacts the epidermis 14, is formed of an expanded, 90 to 120 pore polytetrafluoroethylene material such as materials sold under the trademark Gore-Tex® by W.L. Gore Company of Newark, Delaware or Impragraph sold under the trademark IMPRA® by Impra, Inc. of Tempe, Arizona. The polytetrafluoroethylene portion 20 of flexible sleeve member 12 is normally about 1—8 millimeters in width and preferably remains under the skin line. This compares to a typical epidermis depth of about 0.05 inches. The polytetrafluoroethylene (hereinafter referred to as PTFE) portion 20 extends to where the polyethylene terephthalate portion 22 begins. As an alternative to the use of the polyethylene terephthalate portion 22, a porous titanium coating on body 10 may be used.

Catheter 24 is preferably formed from an approximately 90 centimeter long tube with an inside diameter of approximately 0.080 inches (2 mm) of a medical grade silicone elastomer. Proximal end 26 of catheter 24 is joined to rigid tubular body 10 by forcing the flexible catheter over tubular body ridges 30 formed on tubular body end. Distal end 28 of catheter is trimmed to whatever length is needed to reach the required position within the right auricle.

A closure septum 32 provides an interruptable seal means between the exterior of the body and the body interior. Septum 32 is held in place within tubular access body 10 by a rigid pressure plate 34, which defines an opening 36 therethrough, and by retaining ring 37. Pressure plate 34 is preferably of a molded rigid polymer or titanium.

Preferred septum closure 32 includes a preformed needle opening 38 slit through the septum and extending from near the center of the septum out to the edge thereof. The edges of septum 32 have a groove 40 therein encircling the entire body thereof. Groove 40 carries an elastomeric ring 42 which preferably has an eliptical or circular cross section. Ring 42 is in compression around septum 32 and serves to hold the slit septum together and maintain the slit surfaces together in sealed relationship by applying an inwardly directed radial force on septun 32.

The septum is also preferably provided with an elongated bottom recess and a generally semispherical top recess 46 which is aligned with hole or opening 36 in pressure plate 34 when assembled within body 10. Bottom recess allows for expansion of the septum when a cannula is inserted therethrough. Top recess 46 provides a cannula receiving guide.

Sliding wing 48 which is preferably formed of the same material as flexible sleeve member 12 so as to provide an promote tissue ingrowth is constructed and arranged as shown in Fig. 1 and 2 to slide over catheter 24. Preferably, sliding wing 48 is formed from a one-half inch long (13 mm) by 0.2 inch long (5 mm) by 0.03 inch (0.75 mm) thick sheet of polyethylene terephthalate and includes slits 49 and 50 adjacent the center such that catheter 24 may be passed through one slit and out the other slit. This arrangement allows the wing to be slid easily along catheter 24. The sliding wing is held to the catheter by friction but does not collapse the catheter tube itself.

The carrier cap 52 is formed from a flexible, medical grade silicone elastomer, and is placed over the proximal end of body 10 which extends above epidermis 14. Body 10 preferably includes a raised circumferential lip 53. Carrier cap 52 is preferably constructed and arranged as shown in Figs. 1 and 2 such that a snap fit is accomplished between carrier cap 52 and lip 53 of body 10. Carrier cap 52 is flexible enough to deform and pass over, raised lip 53 of body 10 and seal lip 54 of carrier cap 52 normally holds carrier cap 52 into the snapped position.

An access set 55 includes the carrier cap 52, single probe carrier 56, short shell 64 and access tubing 70. The single probe carrier 56 is preferably formed of a rigid polyvinylchloride polymer which should be molded without the use of silicone as a mold release. The outer circumferential edge 58 of single probe carrier 56 is sized such that it may be snap fitted past seal lip 54 of carrier cap 52 and is thereby held within the carrier cap. A proximally projecting tube 60 formed integrally with single probe carrier 56, serves as the attachment point for access tubing 70. Access tube 70 is preferably and adhesively force fitted over proximally projecting tube 60. Access tubing 70 is then connected to a nutrient source and preferably to a portable infusion pump of various makes.

Single probe carrier 56 is formed with a distal bore 62 which is sized to accept an 11 gauge short shell 64. Short shell 64 is preferably formed of hypodermic grade stainless steel tubing with a closed end 66 and a 0.042 (1.0 mm) inch diameter opening 68 punched through both walls adjacent the closed end 66. Alternatively, short shell 64 may be formed with a hole 68 punched at its rounded end 66. The short shell 64 is of sufficient length such that when the access set 55 is snapped into place over raised lip 53 of body 10, that opening 68 passes through septum 32 such that fluids may be supplied to the catheter.

A sponge 72 is inserted into the excutaneous cavity 74 in body 10 between single probe carrier 56 and retaining ring 37. Sponge 72 is preferably saturated with an antiseptic solution such as the polyvinylpyrolidone/iodine sold umder the trademark Betadine® for maintaining sterility within the cavity 74 of the body 10.

Referring to Fig. 3, a tab cap 76 is shown including an inner lip 78 which may be an integral O-ring which fits over lip 53 of body 10 and provides a hermetic seal. Tab cap 76 preferably is molded of an elastomeric plastic and includes a tab 80 which when grasped by a patient breaks the seal provided to enable the removal of cap 76.

Preferably, whenever nutrients or chemotherapy agents are not being introduced through the device, tab cap 76 is placed over body 10 and disinfectant saturated sponge 72 is sealed within cavity 74 to provide a bacterial barrier and maintain sterility.

In operation, the implant is inserted by a physician into the patient. PTFE portion 20 becomes securely embedded in the epidermis and the remainder of the flexible sleeve member 12 provides further tissue ingrowth to stabilize the implant. Catheter 24 is guided by the physician until it reaches the right auricle. Sliding wing 48 is then maneuvered either visually through a fluoro- scope or by touch until it is embedded deep within the subcutaneous tunnel formed during the implant procedure. The portion of the catheter leading from the sliding wing to the tubular percutaneous body 10 is preferably slightly longer than necessary to travel the distance between wing 48 and body 10. Thus, when sliding wing 48 is embedded subcutaneously in tissue, an outward pull on body 10 merely tends to straighten the catheter up to sliding wing 48 and does not dislodge the distal end of catheter 24 from the right auricle.

The unique design of the PH implant of the invention prevents extrusion of the device by tissue rejection due to its unique flexible sleeve member. The combined action of the polytetrafluoroethylene portion and the polyethylene terephthalate portion provides good embedment. The entire implant is stabilized within the skin and infection is thereby minimized.

## Claims

1. A percutaneous implant device comprising:
a substantially rigid tubular percutaneous body (10) of biologically compatible material which extends tbrough the skin when implanted;
a flexible subcutaneous catheter (24) joined to the tubular percutaneous body (10) for providing fluid comunication between the body exterior and the right auricle of the heart;
carrier cap means (55) including a probe carrier (56) for providing fluid communication from a nutrient supply tube to said catheter;
and porous tissue ingrowth means (12,48) for anchoring the device to the body;
is characterised in that the porous tissue ingrowth means comprise:
a tissue ingrowth (12) sleeve comprising a porous collar of polytetrafluoroethylene (20) overlaying the portion of the tubular percutaneous body (10) to be embedded in the epidermis and an adjacent porous collar of polyethylene terephthalate (22) being of sufficient length to match the thickness of the dermis;
and a sliding wing (48) of porous tissue ingrowth material encircling part of, and slidably positionable on, the subcutaneous catheter.

2. A device in accordance with claim 1 wherein the sliding wing (48) of porous tissue ingrowth material is of polyethylene terephthalate.

3. A device in accordance with claim 1 or 2 wherein the sliding wing (48) projects outwardly.

4. A device in accordance with any preceding claim wherein said tubular body includes in the interior thereof an elastomeric septum (32) and means (42) for holding said septum in a sealing relationship therein.

5. A device in accordance with claim 4 wherein said elastomeric septum (32) includes an elongate recess on the surface facing said catheter (24) and a centred conical depression (46) on the surface facing away from said catheter.

6. A device in accordance with claim 4 or 5 wherein said elastomeric septum (32) includes a preformed slit (38) therethrough and in which the septum is surrounded by an elastomeric O-ring (42) such that the slit is closed due to radial pressure from said O-ring.

7. A device in accordance with claim 6 wherein the carrier cap means (55) includes a carrier cap (52) constructed and arranged to snap fit over said tubular percutaneous body (10), the probe carrier (56) extending through said cap, said cap including nutrient tubing connection means (60) and a short shell cannula (64) for penetrating said septum when said cap is secured to the tubular body.

## Patentansprüche

1. Perkutane Implantierungsvorrichtung, aufweisend:
einen im wesentlichen starren röhrenartigen perkutanen Körper (10) aus biologisch verträglichem Material, der sich, implantiert, durch die Haut erstreckt;
einen flexiblen subkutanen Katheter (24), der mit dem röhrenartigen perkutanen Körper (10) zur Schaffung einer Fluidkommunikation zwischen dem Äußeren des Körpers und dem rechten Vorhof des Herzens verbunden ist;
eine Trägerkappeneinrichtung (55), einschließend einen Sondenträger (56) zur Schaffung einer Fluidkommunikation von einer Nährstoffzuleitungsröhre zum Katheter;
und eine poröse Gewebeeinwachseinrichtung (12, 48) zum Verankern der Vorrichtung am Körper;
ist dadurch gekennzeichnet, daß die poröse Einwachseinrichtung aufweist:
eine Gewebeeinwachsmanschette (12), die einen porösen Manschettenrand aus Polytetrafluorethylen (20), der über dem in die Epidermis einzubettenden Abschnitt des röhrenartigen perkutanen Körpers (10) liegt, und einen angrenzenden porösen Manschettenrand aus Polyethylenterephthalat (22) umfaßt, der eine zur Anpassung an die Dicke der Haut ausreichende Länge hat;
und einen verschiebbaren Flügelansatz (48) aus porösem Gewebeeinwachsmaterial, der einen Teil des subkutanen Katheters, auf dem er durch gleitende Verschiebung positionierbar ist, umschließt.

2. Vorrichtung nach Anspruch 1, in welcher der verschiebbare Flügelansatz (48) aus porösem Gewebeeinwachsmaterial aus Polyethylenterephthalat besteht.

3. Vorrichtung nach Anspruch 1 oder 2, in welcher der verschiebbare Flügelansatz (48) nach außen übersteht.

4. Vorrichtung nach einem der vorhergehenden Ansprüche, in welcher der röhrenartige Körper in seinem Innern ein Elastomer-Septum (32) und eine Einrichtung (42) enthält, um das Septum darin in abdichtender Relation zu halten.

5. Vorrichtung nach Anspruch 4, in welcher das Elastomer-Septum (32) eine längliche Aussparung auf der dem Katheter (24) gegenüberliegenden Fläche und eine zentrische konische Vertiefung (46) auf der vom Katheter abgewandten Fläche aufweist.

6. Vorrichtung nach Anspruch 4 oder 5, in welcher das Elastomer-Septum (32) eine vorgeformte Nut (38) durch das Septum aufweist und in welcher das Septum von einem Elastomer-O-Ring (42) derart umgeben ist, daß die Nut infolge des radialen Drucks vom O-Ring geschlossen wird.

7. Vorrichtung nach Anspruch 6, in welcher die Trägerkappeneinrichtung (55) eine Trägerkappe (52) aufweist, die so aufgebaut und angeordnet ist, daß sie über dem röhrenartigen perkutanen Körper (10) passend festschnappt, daß der Sondenträger (56) sich durch diese Kappe erstreckt und daß die Kappe eine Nährstoffröhrenzuleitungsanschlußeinrichtung (60) und eine kurze Hohlkanüle (64) zum Durchdringen des Septums, wenn die Kappe am röhrenartigen Körper befestigt wird, aufweist.

**Revendications**

1. Dispositif à implant percutané, comprenant:
un corps tubulaire percutané sensiblement rigide (10) d'un matériau biologiquement compatible, passant à travers de la peau lorsqu'il est implanté,
un cathéter sous-cutané souple (24) raccordé au corps tubulaire percutané (10) et destiné à assurer la communication entre l'extérieur du corps et l'oreillette droite du coeur,
un capuchon (55) de support comprenant un support (56) de sonde destiné à assurer la communication entre un tube d'alimentation en matières nutritives et le cathéter, et
un dispositif d'incarnation à tissu poreux (12, 48) destiné à assurer l'ancrage du dispositif sur le corps,
caractérisé en ce que le dispositif de croissance à tissu poreux comprend:
un manchon (12) de croissance de tissu comprenant un collier poreux de polytétrafluoréthylène (20) recouvrant la partie du corps tubulaire percutané (10) qui doit être enrobée dans l'épiderme et un collier poreux adjacent de téréphtalate de polyéthylène (22) ayant une longueur suffisante pour qu'il corresponde à l'épaisseur du derme, et
une ailette coulissante (48) formée d'un matériau de croissance à tissu poreux, entourant une partie souscutanée du cathéter et pouvant être déplacée par coulissement sur ce cathéter.

2. Dispositif selon la revendication 1, dans lequel l'ailette coulissante (48) en matériau de croissance à tissu poreux est formée de téréphtalate de polyéthylène.

3. Dispositif selon la revendication 1 ou 2, dans lequel l'ailette coulissante (48) dépasse vers l'extérieur.

4. Dispositif selon l'une quelconque des revendications précédentes, dans lequel le corps tubulaire a, à l'intérieur, une cloison élastomère (32) et un dispositif (42) de maintien de la cloison en coopération étanche avec lui.

5. Dispositif selon la revendication 4, dans laquelle la cloison élastomère (32) a une cavité allongée à la surface tournée vers le cathéter (24) et une cavité conique centrée (46) à la surface opposée au cathéter.

6. Dispositif selon la revendication 4 ou 5, dans lequel la cloison élastomère (32) a une fente (38) préalablement formée qui la traverse, et dans lequel la cloison est entourée par un joint torique élastomère (42) de manière que la fente soit fermée par la pression radiale exercée par le joint torique.

7. Dispositif selon la revendication 6, dans lequel le capuchon de support (55) comporte un capuchon (52) dont la construction et la disposition sont telles qu'il s'enclenche élastiquement sur le corps tubulaire percutané (10), le support de sonde (56) traversant le capuchon, le capuchon ayant un dispositif (60) de raccordement d'un tube de matières nutritives et une canule (64) à courte enveloppe destinée à traverser la cloison lorsque le capuchon est fixé au corps tubulaire.

Fig. 1

Fig. 2

Fig. 3